Europäisches Patentamt

European Patent Office

Office européen des brevets

(11) Numéro de publication: **0 254 793**
**A1**

## (12) DEMANDE DE BREVET EUROPEEN

(21) Numéro de dépôt: 86870105.3

(22) Date de dépôt: 31.07.86

(51) Int. Cl.⁴: **G01N 1/28** , G01N 33/48 , A61B 10/00

(43) Date de publication de la demande:
**03.02.88 Bulletin 88/05**

(84) Etats contractants désignés:
**AT BE CH DE FR GB IT LI LU NL SE**

(71) Demandeur: **SOCIETE SANDERS PROBEL BIOTECHNOLOGY, société anonyme rue de Wallons, 6 Louvain-la-Neuve(BE)**

(72) Inventeur: **Havaux, Jean-Claude 4, avenue des Renards B-4040 Tilff(BE)**

(74) Mandataire: **Vigneron, Jean Cabinet VIGNERON 30 avenue Eugène Godaux B-1150 Bruxelles(BE)**

(54) **Dispositif pour le prélèvement dans une masse de matière, d'un volume déterminé de cette matière.**

(57) Dispositif (1) pour le prélèvement, dans une masse de matière (2) , d'un volume déterminé (3) de celle-ci , comprenant une enceinte (4) ouverte à sa base (5) et dont le volume interne libre correspond au volume (3) de l'échantillon à prélever, un fourreau (9) enserrant les parois externes (8) de l'enceinte de manière à pouvoir coulisser librement sur ces parois (8) , des moyens (10) agencés sur ces dernières pour prendre appui sur le bord (11) du fourreau afin d'empêcher le coulissement de celui-ci sur l'enceinte quand sa base est introduite dans la matière et des moyens (14), répartis à l'extérieur du fourreau, agencés pour coopérer avec la matière pour l'y maintenir quand l'enceinte (4) est extraite de la matière.

FIG.1.

## Dispositif pour le prélèvement dans une masse de matière, d'un volume déterminé de cette matière.

La présente invention a pour objet un dispositif pour le prélèvement , dans une masse de matière, d'un volume déterminé de cette matière, en particulier pour le prélèvement d'un échantillon de selles.

L'invention a pour but de procurer un dispositif de prélèvement, du type susdit, qui offre le grand avantage, lorsqu'il est extrait de la matière après prélèvement, de présenter toutes les parties externes totalement exemptes de matière, ce qui est particulièrement intéressant sur divers plans, à savoir : facilité et agrément de l'emploi, propreté de l'emploi et prélèvement exact du volume déterminé.

A cet effet, suivant l'invention, ledit dispositif comprend une enceinte ouverte à sa base, dont au moins la section externe est constante, dont les faces internes et externes sont lisses et dont le volume interne libre correspond au volume de l'échantillon à prélever, un fourreau enserrant les parois externes de l'enceinte, à partir de la base cette dernière et sur une hauteur au moins égale à la hauteur interne de l'enceinte, de manière à pouvoir coulisser sur ces parois externes,des moyens agencés sur ces dernières pour prendre appui sur le bord du fourreau, opposé au bord de ce dernier situé au niveau de la base de l'enceinte, afin d'empêcher le coulissement du fourreau sur l'enceinte lorsque la base de cette dernière est introduite dans la matière et des moyens, régulièrement répartis à l'extérieur dudit fourreau, agencés de telle sorte qu'ils coopèrent avec la matière pour maintenir ledit foureau dans la matière lorsque l'enceinte précitée est extraite de cette matière.

Suivant une forme de réalisation avantageuse de l'invention, ledit dispositif comprend des moyens agencés pour dégager l'échantillon prélevé de l'enceinte.

Suivant un mode de réalisation particulièrement avantageux de l'invention, les parois externes de l'enceinte comprennent, dans leur zone non recouverte par le fourreau, des moyens pour fixer ladite enceinte à l'intérieur d'un récipient ou sur une colonne d'analyse.

D'autres détails et particularités de l'invention ressortiront de la description des dessins annexés au présent mémoire et qui représentent, à titre d'exemples non limitatifs, diverses formes de réalisation du dispositif de prélèvement suivant l'invention.

La figure 1 est une vue en élévation et en coupe du dispositif de prélèvement suivant l'invention, celui-ci étant représenté plongé dans la matière pour effectuer le prélèvement.

La figure 2 est une vue analogue à la figure 1 et montre le dispositif en cours de dégagement de la matière après prélèvement de l'échantillon.

La figure 3 est une vue en élévation, avec brisures partielles, d'une variante du dispositif de prélèvement illustré aux figures 1 et 2.

La figure 4 est une vue en élévation, partiellement en coupe, montrant le dispositif illustré à la figure 3 fixé à l'intérieur d'un récipient.

La figure 5 est une vue en élévation, avec brisures partielles, d'une autre variante du dispositif de prélèvement illustré aux figures 1 et 2.

La figure 6 est une vue en élévation, avec brisures partielles, montrant le dispositif illustré à la figure 5 fixé sur une colonne de réaction.

Dans les différentes figures, les mêmes notations de référence désignent des éléments identiques ou analogues.

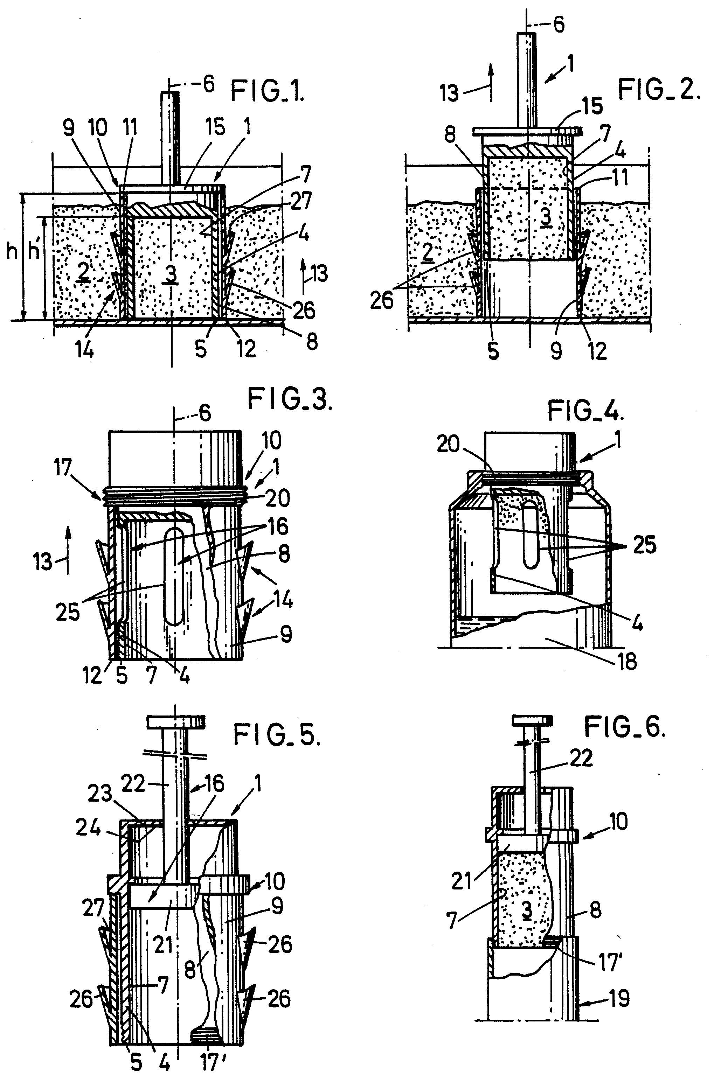

Le dispositif 1, suivant l'invention et illustré aux dessins, est destiné au prélèvement,dans une masse de matière 2 ( figures 1 et 2) , d'un volume déterminé 3 de cette matière. Ce dispositif, qui est tout particulièrement destiné au prélèvement d'échantillons de selles, comprend une enceinte 4, ouverte à sa base 5, dont les sections interne et externe, prises perpendiculairement à l'axe 6 de l'enceinte, sont constantes, dont les faces internes 7 et externes 8 sont lisses et dont le volume interne libre correspond au volume 3 de l'échantillon de matière à prélever.

Ledit dispositif 1 comprend également un fourreau 9 enserrant les parois externes 8 de l'enceinte 4, à partir de la base 5 de celle-ci et sur une hauteur h au moins égale à la hauteur interne h' de l'enceinte 4 , de telle sorte qu'il puisse coulisser sur lesdites parois externes. Des moyens 10 sont agencés sur ces parois 8 pour prendre appui sur le bord 11 du fourreau 9, qui est opposé au bord 12 de ce dernier situé au niveau de la base 5 de l'enceinte 4, afin d'empêcher le coulissement dudit fourreau sur l'enceinte, suivant la flèche 13, lorsque la base 5 de cette enceinte 4 est introduire dans la matière 2,tandis que des moyens 14 , régulièrement répartis à l'extérieur du fourreau 9, sont agencés de telle sorte qu'ils coopèrent avec la matière 2 pour maintenir le fourreau 9 dans la matière quande l'enceinte 4 est extraite de celle-ci.

Il ressort clairement de la figure 1 que les moyens 10, constitués par un rebord périphérique 15 présenté par l'enceinte 4, maintient le fourreau 9 en position sur les parois externes 8 de l'enceinte 4 lorsque le dispositif est enfoncé dans la matière 2, de sorte qu'aucune trace de matière ne peut se déposer sur ces parois externes 8. Lors du retrait du dispositif de la matière, avec l'échantillon à prélever contenu dans l'enceinte, on remarque, à la figure 2, que le fourreau, retenu dans la matière par les moyens 14, coulisse librement sur lesdites parois externes de l'enceinte et isole totalement ces dernières de la matière, de sorte qu'on maintient une propreté parfaite de ces parois externes 8.

Le dispositif 1 suivant l'invention comprend avantageusement , comme montré aux figures 3 à 6, des moyens 16 agencés pour dégager l'échantillon prélevé de l'enceinte 4.

Suivant l'invention, ledit dispositif 1 peut également avantageusement comprendre soit, comme montré aux figures 3 et 4, des moyens 17 pour fixer ladite enceinte 4 à l'intérieur d'un récipient 18 afin d'assurer le transport et le stockage de l'échantillon prélevé parfaitement isolé, soit,comme montré aux figures 5 et 6, des moyens 17' pour fixer l'enceinte 4 sur une colonne de réaction 19, ce qui permet une analyse de l'échantillon par transfert direct de ce dernier de l'enceinte 4 dans la colonne de réaction 19.

Comme montré aux figures 3 à 6 , les moyens 17 et 17' susdits sont constitués par un pas de vis 20 réalisé à l'extérieur de l'enceinte 4 et destiné à coopérer avec un pas de vis interne prévu sur le récipient 18 ou la colonne de réaction 19. Dans la forme de réalisation du dispositif 1 illustrée aux figures 3 et 4, ce pas de vis 20 constitue également les moyens 10 prenant appui sur le bord 11 du fourreau 9 afin de s'opposer au déplacement de ce dernier suivant la flèche 13.

Dans la forme de réalisation du dispositif 1 montrée aux figures 5 et 6 , les parois de l'enceinte 4 sont pleines, les moyens 16 précités, prévus pour dégager l'échantillon prélevé de l'enceinte 4, sont constitués par un piston 21 logé dans l'enceinte 4 et destiné à coopérer avec les parois internes 7 de cette dernière. Ce piston 21 est actionné par une tige de piston 22 qui fait saillie par rapport à la paroi 23 de l'enceinte, cette tige coulissant, pour être guidée suivant l'axe 6 dans une ouverture 24 prévue dans cette paroi 23.

Dans la forme de réalisation du dispositif 1 illustrée aux figures 3 et 4, les parois de l'enceinte 4 présentent, dans leurs parties destinées à être recouvertes par le fourreau 9, des ouvertures 25 régulièrement réparties sur le pourtour de ladite enceinte 4. Les moyens 16 précités pour dégager l'échantillon prélevé de l'enceinte sont dans ce cas constitués par un liquide contenu dans le récipient 18 et qui est destiné à diluer l'échantillon.

Les moyens 14, prévus à l'extérieur du fourreau 9 pour maintenir ce dernier dans la matière lors du retrait de l'enceinte 4 de cette matière, sont constitués par des aspérités 26 qui s'étendent transversalement par rapport au fourreau. Ces aspérités sont avantageusement, comme montré aux dessins, constituées par des éléments profilés faisant corps avec le fourreau 9 pour former des alvéoles dont les ouvertures sont opposées à la base 5 de l'enceinte 4.

Il doit être entendu que l'invention n'est nullement limitée aux formes de réalisation décrites et que bien des modifications peuvent être apportées à ces dernières sans sortir du cadre du présent brevet.

## Revendications

1) Dispositif (1) pour le prélèvement, dans une masse de matière (2), d'un volume déterminé (3) de cette matière, en particulier pour le prélèvement d'un échantillon de selles, caractérisé en ce qu'il comprend une enceinte (4) ouverte à sa base (5) , dont au moins la section externe est constante, dont les faces internes (7) et externes (8) sont lisses et dont le volume interne libre correspond au volume (3) de l'échantillon à prélever, un fourreau (9) enserrant les parois externes (8) de l'enceinte (4), à partir de la base (5) de cette dernière et sur une hauteur (h) au moins égale à la hauteur interne (h') de l'enceinte (4), de manière à pouvoir coulisser sur ces parois externes (8) , des moyens (10) agencés sur ces dernières pour prendre appui sur le bord (11) du fourreau (9) , opposé au bord (12) de ce dernier situé au niveau de la base (5) de l'enceinte (4) , afin d'empêcher le coulissement du fourreau (9) sur l'enceinte lorsque la base (5) de cette dernière est introduite dans la matière (2) et des moyens (14), régulièrement répartis à l'extérieur dudit fourreau (9), agencés de telle sorte qu'ils coopèrent avec la matière (2) pour maintenir ledit fouureau (9) dans la matière lorsque l'enceinte (4) précitée est extraite de cette matière.

2) Dispositif suivant la revendication 1, caractérisé en ce qu'il comprend des moyens (16) agencés pour dégager l'échantillon prélevé de l'enceinte (4).

3) Dispositif suivant l'une ou l'autre des revendications 1 et 2, caractérisé en ce que les parois externes (8) de l'enceinte (4) comprennent, dans leur zone non recouverte par le fourreau (9), des moyens (17, 17') pour fixer ladite enceinte (4) à l'intérieur d'un récipient (18) ou sur une colonne de réaction(19).

4) Dispositif suivant la revendication 3, caractérisé en ce que les moyens (17, 17') destinés à fixer l'enceinte (4) à l'intérieur d'un récipient sont constitués par un pas de vis (20) réalisé à l'extérieur de l'enceinte (4) et destiné à coopérer avec un pas de vis interne prévu sur le récipient (18) ou la colonne de réaction (19).

5) Dispositif suivant l'une ou l'autre des revendications 3 et 4 , caractérisé en ce que les moyens (17, 17') destinés à fixer l'enceinte à l'intérieur d'un récipient constituent les moyens (10) précités agencés pour prendre appui sur le bord (11) du fourreau (9) opposé au bord (12) de ce dernier situé au niveau de la base (5) de l'enceinte (4).

6) Dispositif suivant l'une quelconque des revendications 2 à 5 , caractérisé en ce que les parois de l'enceinte (4) sont pleines, les moyens (16) précités agencés pour dégager l'échantillon prélevé étant constitués par un piston (21) logé dans l'enceinte (4) et destiné à coopérer avec les parois internes (7) de cette dernière, la tige (22) du piston faisant saillie par rapport à la paroi (23) de l'enceinte opposée à la base de celle-ci et étant guidée dans cette paroi.

7) Dispositif suivant l'une quelconque des revendications 3 à 5 , caractérisé en ce que les parois de l'enceinte (4) destinées à être recouvertes par le fourreau (9) présentent des ouvertures (25) régulièrement réparties sur le pourtour de l'enceinte et destinées à être situées à l'intérieur du récipient (18) sur lequel est fixée l'enceinte, les moyens (16) précités agencés pour dégager l'échantillon prélevé de l'enceinte étant constitués par un liquide contenu dans ledit récipient (18) et destiné à diluer l'échantillon.

8) Dispositif suivant l'une quelconque des revendications 1 à 7 , caractérisé en ce que les moyens (14) , prévus à l'extérieur du fourreau (9) pour maintenir ce dernier dans la matière lors du retrait de l'enceinte de celle-ci, sont constitués par des aspérités (26) s'étendant transversalement par rapport au fourreau.

9) Dispositif suivant la revendication 8, caractérisé en ce que les aspérités (26) susdites sont constituées par des éléments profilés assemblés au fourreau (9) pour former des alvéoles dont les ouvertures sont opposées à la base (5) de l'enceinte (4).

FIG_1.

FIG_2.

FIG_3.

FIG_4.

FIG_5.

FIG_6.

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | DE-A-3 427 114 (SZABADOS) * en entier * | 1 | G 01 N 1/28 G 01 N 33/48 A 61 B 10/00 |
| | --- | | |
| A | US-A-4 293 405 (R.J. GREENWALD) * figures; revendications * | 1 | |
| | --- | | |
| A | US-A-4 318 803 (R.S. HOLMGREN) * figures; revendications * | 1 | |
| | --- | | |
| A | EP-A-0 076 507 (BOEHRINGER MANNHEIM GMBH) | | |
| | ----- | | |

### DOMAINES TECHNIQUES RECHERCHES (Int. Cl.4)

G 01 N 1/00
G 01 N 33/00
A 61 B 10/00
B 01 L 3/00

Le présent rapport de recherche a été établi pour toutes les revendications

| Lieu de la recherche | Date d'achèvement de la recherche | Examinateur |
|---|---|---|
| BERLIN | 16-02-1987 | VINSOME R M |